(19) Europäisches Patentamt
European Patent Office
Office européen des brevets

(11) Publication number: **0 100 471**
**B1**

(12) # EUROPEAN PATENT SPECIFICATION

(45) Date of publication of patent specification: **22.10.86**

(51) Int. Cl.$^4$: **C 07 D 311/72**

(21) Application number: **83106863.0**

(22) Date of filing: **13.07.83**

(54) **Alpha tocopherol process.**

(30) Priority: **29.07.82 US 403085**
**30.09.82 US 430154**

(43) Date of publication of application:
**15.02.84 Bulletin 84/07**

(45) Publication of the grant of the patent:
**22.10.86 Bulletin 86/43**

(84) Designated Contracting States:
**CH DE FR GB LI**

(56) References cited:
**EP-A-0 012 824**
**EP-A-0 058 945**
**EP-A-0 087 576**

**CHEMICAL ABSTRACTS, vol. 80, no. 11, March 18, 1974, Columbus, Ohio, USA A.A. SVISHCHUK, E.D. BASALKEVICH, N.N. VYSOTSKII, E.D. OVERCHUK "Synthesis of DL-alpha-tocopheryl acetate (vitamin E)" page 347, column 1, abstract no. 59 820x**

(73) Proprietor: **BASF WYANDOTTE CORPORATION**
**1609 Biddle Avenue**
**Wyandotte Michigan 48192 (US)**

(72) Inventor: **Finnan, Jeffrey Lawrence**
**13656 Edison Blvd.**
**Southgate Michigan 48192 (US)**

(74) Representative: **Schweiss, Werner, Dr. et al**
**BASF Aktiengesellschaft Patentabteilung Carl-Bosch-Strasse 38**
**D-6700 Ludwigshafen (DE)**

(56) References cited:

**CHEMICAL ABSTRACTS, vol. 93, no. 7, August 18, 1980, Columbus, Ohio, USA DESAI, "Assay methods for tocopherols" page 410, column 2, abstract no. 64 902y**

**CHEMICAL ABSTRACTS, vol. 81, no. 3, July 22, 1974, Columbus, Ohio, USA FERENC GERLOCZY, "Tocopherol (vitamin E), Present state of research new results" page 146, column 2, abstract no. 11 116r**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 100 471 B1

**Description**

This invention relates to an improved process for manufacturing d,l-alpha-tocopherol.

The preparation of d,l-alpha-tocopherol by the condensation of trimethylhydroquinone and a phytyl derivative in the presence of an inert solvent and acid condensing agents is well known in the art. The acid catalyst can be either or both a Lewis acid such as zinc chloride or a strong inorganic acid such as hydrochloric acid, sodium bisulfate or para-toluenesulfonic acid. In addition, it is known from U.S. 4,191,692 to pre-react isophytol with a small amount of ammonia or an amine prior to reaction with trimethylhydroquinone.

The use of zinc chloride, an inert solvent and gaseous hydrochloric acid in the process of producing d,l-alpha-tocopherol is disclosed by Karrer in U.S. 2,411,967; U.S. 2,411,968; and U.S. 2,411,969. Greenbaum et al in U.S. 3,708,505 also disclose the use of a combination of a Lewis acid and a strong acid in a process for the preparation of d,l-alpha-tocopherol.

Improved process catalysts or combinations thereof are also disclosed in U.S. 4,208,334; 2,723,278; 3,789,086; 3,459,773; 3,444,213; and 4,217,285.

It has been unexpectedly discovered that by including a small amount of an amine salt of a non-oxidizing protic acid or a amine thus forming in situ the amine salt, in the reaction mixture for the preparation of d,l-alpha-tocopherol prior to the addition of the phytyl derivative that an improved product is obtained, specifically a product having greater purity. Additionally, the percent yield is also increased. In the process of the invention, trimethylhydroquinone and an inert organic solvent are charged into a reaction vessel together with a combination of at least one Lewis acid, at least one strong acid, and at least one of said amine or amine salt. A phytyl derivative such as isophytol is then slowly added to the reaction mixture and when addition has been completed, the reaction mass is refluxed for a sufficient time to produce d,l-alpha-tocopherol. Optionally, acetic anhydride can be added to produce d,l-alpha-tocopherol acetate. It is a particular advantage of the present process that the synthesis is essentially a one-step process which produces a higher yield as well as a higher quality d,l-alpha-tocopherol.

The phytyl derivative useful as a reactant in the process of the invention has the formulas:

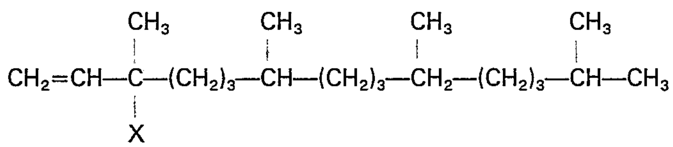

(I)

and

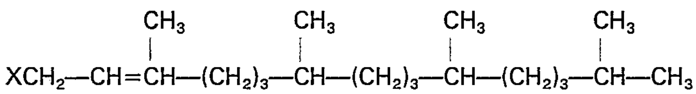

(II)

wherein X is a leaving group. Useful leaving groups include hydroxy, halogen, alkylsulfonyloxy, arylsulfonyloxy, alkoxy, and alkanoyloxy all with 1 to 7 carbon atoms. The halogen-leaving groups are preferably chlorine and bromine. The preferred alkylsulfonyloxy leaving group is mesyloxy. The preferred arylsulfonyloxy leaving group is tosyloxy. The preferred alkanoyloxy group is acetoxy. The preferred alkoxy leaving groups are n-butoxy, methoxy, isobutoxy, and ethoxy.

As used throughout the specification, the term "halogen" includes all four halogens such as bromine, chlorine, fluorine and iodine.

The term "alkyl containing 1 to 7 carbon atoms" includes particularly methyl, ethyl, propyl, isopropyl and isobutyl.

The term "alkoxy containing from 1 to 7 carbon atoms" includes particularly methoxy, ethoxy, n-butoxy and isobutoxy.

The term "alkanoyl containing from 1 to 7 carbon atoms", preferably from 2 to 7 carbon atoms includes particularly acetyl and propionyl.

The terms "phenyl" and "naphthyl" denote radicals which can be unsubstituted or substituted in one or more positions with a lower alkyl or nitro group.

Specific examples of phytyl derivatives useful in the preparation of the alpha-tocopherols of the invention include phytol, isophytol, phytadiene, phytyl chloride, phytyl bromide, phytyl acetate and phytyl methyl ether. The preferred phytyl derivative is isophytol.

The inert organic solvents which can be employed as a reaction medium in the process of the invention are those aromatic and aliphatic hydrocarbon solvents having a boiling point below the boiling points of the reactants and the desired product. Preferred solvents include aliphatic hydrocarbon solvents having about 5 to about 12 carbon atoms. Representative aromatic hydrocarbon solvents include xylene, benzene, and toluene. Miscellaneous inert organic solvents also can be used. Representative aliphatic hydrocarbon solvents include pentane, hexane, heptane, octane, nonane, decane, undecane, and dodecane. Preferably, n-heptane is utilized. Other inert organic solvents can be used such as an aliphatic ether such as isopropyl ether.

The Lewis acids useful as catalysts in the process of the invention include boron trifluoride, boron

tribromide, aluminum chloride, aluminum bromide, zinc chloride, boron trifluorodiphosphoric acid complex. The preferred Lewis acid is zinc chloride.

Strong acid catalysts useful in the process of the invention include hydrogen chloride gas, sulfuric acid, hydrochloric acid, nitric acid, para-toluene sulfonic acid, sodium bisulfate, and mixtures thereof. Preferably, strong acids such as sulfuric acid, para-toluene sulfonic acid, and sodium bisulfate are used. Especially preferred is anhydrous hydrogen chloride gas present in the reaction mixture in an amount sufficient to saturate said reaction mixture.

The amines which are useful by direct addition or in the formation of the amine salts to be added to the process of the invention are arylaliphatic amines having 7 to 24 carbon atoms or aliphatic and cycloaliphatic amines having 7 to 24 carbon atoms, preferably 18 to 22 carbon atoms, in the aliphatic chain. The amines can be primary, secondary or tertiary amines. The alkyl primary monoamines which can have straight or branched chains are preferred. Generally, the amine as such or preferably in the form of a salt, is used in the process of the invention in an amount of 0.05 percent to 5 percent by weight, preferably 0.1 percent to 2 percent by weight, all based upon the weight of the phytyl derivative used in the process. Representative examples of useful alkyl amines are tridecylamine, 1-docosanamine, 1-eicosanamine, pentadecylmethylamine, octadecyldimethylamine and dioctyldecylamine. A further example of a useful cycloaliphatic amine is cyclohexylamine. A representative example of a useful arylaliphatic amine is benzylamine. Amines useful in the process of the invention can be further substituted with groups such as hydroxy, lower alkoxy, or lower alkylamine groups.

The non-oxidizing, protic acids useful in the formation of the amine salts of the invention can be inorganic or organic acids generally having a pK of up to 5, preferably 0.5 to 5, most preferably 1 to 5. Protic acids are defined as those acids which are capable of giving up a proton in a reaction with a basic material. Useful acids include the following acids:

| | |
|---|---|
| acetic | 2,4-dinitrophenol |
| adipic | diphenylacetic |
| ascorbic | ethylbenzoic |
| barbituric | ethylphenylacetic |
| benzoic | formic |
| benzosulfonic | fumaric |
| n-butyric | gallic |
| n-caproic | glutaramic |
| citric | glutaric |
| crotonic | glycolic |
| dihydroxybenzoic | heptanoic |
| dihydroxymalic | hexahydrobenzoic |
| dihydroxytartaric | a-hydroxybenzoic |
| dimethylmalic | a-hydroxypropionic |
| dimethylmalonic | itaconic |
| maleic | malic |
| lactic | malonic |
| naphtholenesulfonic | mesaconic |
| a-nitrobenzoic | oxalic |
| phenylacetic | phenylbenzoic |
| phenylbutyric | paratoluenesulfonic |
| phenylpropionic | hydrofluoric |
| phthalic | phosphoric |
| propionic | phosphorus |
| suberic | pyrophosphoric |
| succinic | selenic |
| sulfanilic | selenious |
| a-tartaric | sulfuric |
| thioacetic | sulfurous |
| vinylacetic | methanesulfonic |

The amine salts to be used in the invention can be obtained by combination of the above-defined amine and said non-oxidizing protic acid under suitable reaction conditions such as ambient or elevated temperature and pressure for 15 minutes to 2 hours and recovering the amine salt reaction product. Reaction conditions are conventional and known to those skilled in this art thus obviating the need for further disclosure herein.

The general reaction of the phytyl derivative with trimethylhydroquinone is known in the art, as indicated by the procedures provided in U.S. 3,708,505, incorporated herein by reference. The process of the invention is performed by charging trimethylhydroquinone into a reaction flask together with the Lewis acid, strong acid, amine or amine salt, and inert organic solvent. The combination is then heated at a temperature of 50°C to about 150°C and the phytyl derivative is added slowly to said combination of

ingredients over a period of about 2 to about 6 hours. The reaction mass is then further heated at about 50°C to about 150°C for about 6 to about 24 hours. When the reaction is completed, the mass can be purified by distillation if so desired.

As compared to the process of U.S. 4,191,692 one of the useful and unexpected results of adding to the reaction mixture a small amount of an amine or preferably an amine salt in combination with the Lewis acid and strong acid prior to the addition of the phytyl derivative in accordance with the process of the invention is that the d,l-alpha-tocopherol produced has a purity of 94.8 to 95.6 percent by weight, which meets feed grade standards. Purification is, therefore, only required if the product is to meet national formulary standards. Alternatively, acetic anhydride can be added to the crude d,l-alpha-tocopherol obtained by the process of the invention in order to convert the tocopherol to d,l-alpha-tocopherol acetate.

The ratio of reactants is from 0.9 to 1.1 mole, preferably 1.0 mole, of trimethylhydroquinone to 0.9 to 1.1 mole, preferably 1.0 mole, of phytyl derivative (preferably isophytol) in combination with 0.1 to 1.5 moles each, preferably 0.35 to 0.75 mole, of the Lewis acid and strong acid catalyst. The amount of inert organic solvent present in the reaction mixture can vary, the minimal proportion necessary being that amount sufficient for the reaction to take place. Thus, the inert organic solvent can be present in the proportion of from 1 to 10 parts by weight per part by weight of the phytyl derivative.

The amount of Lewis acid or strong acid catalyst can be from 0.04 part by weight, per part by weight of the phytyl derivative, up to very large amounts of 0.5 part by weight of the phytyl derivative or more although large amounts provide no advantage.

The strong acid which is used can be an aqueous acid such as concentrated hydrochloric acid and concentrated hydrobromic acid or strong mineral acids such as sulfuric acid or sodium bisulfate. Hydrochloric acid is preferred, and it is especially preferred that instead of aqueous hydrochloric acid, that hydrogen chloride gas be employed. This can be passed into the reaction mixture during the reaction and maintained at a saturation concentration. The use of hydrogen chloride gas has the particular advantage that the acid concentration cannot rise excessively since hydrogen chloride gas present in excess of that required to saturate the reaction mixture volatilizes from the reaction mixture.

The following examples illustrate the various aspects of the invention.

Where not otherwise specified throughout this specification and claims, temperatures are given in degrees centigrade and parts, percentages, and proportions are by weight.

Example 1
(Control, forming no part of this invention)

In this example, and in Example 2, the process of the prior art is shown in which isophytol is mixed and prereacted with an amine prior to reaction with trimethylhydroquinone.

Crude isophytol (97.5 percent by weight purity) in the amount of 100 parts by weight was mixed with 0.3 part by weight of tridecylamine and thereafter heated at 80°C for two hours. After cooling, the product of the isophytylamine reaction was used directly in the condensation reaction with trimethylhydroquinone as described below.

The amine-isophytol reaction product was added dropwise to a mixture of trimethylhydroquinone (76.1 grams, 0.495 mole) zinc chloride 95 percent by weight aqueous (20.7 grams, 0.150 mole) in technical grade n-heptane (380 ml). The mixture was heated to constant reflux with a continuous hydrogen chloride sparge and the amine-isophytol reaction product (181 ml, 0.50 mole) was added dropwise over a period of 90 minutes. After completion of the addition of the amine-treated isophytol, the hydrogen chloride sparge was continued for an additional 15 minutes, and five minutes later the heating was stopped. After cooling for one hour, the reaction mixture was extracted with three portions (350 ml) of methanol/water (1:1), acidified with 1 ml of concentrated hydrochloric acid, and the heptane was evaporated under vacuum. The crude tocopherol was then reacted with acetic anhydride (200 ml), 2.1 mole for a period of four hours at reflux temperature. The resulting acetic acid and excess anhydride were removed under vacuum. The crude alpha-tocopherol acetate was assayed via gas chromatography to determine the yield in purity. The results are indicated in the following table.

Example 2
(Control, forming no part of this invention)

Example 1 was repeated except that tridecyl amine was utilized to treat the isophytol by mixing one weight percent of said amine with 100 parts by weight of the crude isophytol of Example 1 and thereafter heating at 80°C for two hours. The amine-treated isophytol was utilized in the same proportions and the same procedure was followed as described in Example 1.

Example 3

Under a nitrogen atmosphere there were added to a reaction container 380 ml of technical grade n-heptane, 99.0 percent by weight trimethylhydroquinone (76.1 grams, 0.495 mole), 95 percent by weight zinc chloride (20.7 grams, 0.150 mole) and 0.3 weight percent of octadecylamine acetate based upon the weight of the crude isophytol to be added subsequently, namely, 181 ml, (0.50 mole) of 97.5 percent isophytol. The mixture obtained was heated to constant reflux with a continuous hydrogen chloride sparge and then the isophytol was added over a 90 minute period. After completion of the isophytol addition, the

hydrogen chloride sparge was continued for an additional 15 minutes, and five minutes later the heating was stopped. After cooling for a period of one hour, the reaction mixture was extracted with three portions (350 ml) of methanol/water (1:1) acidified with 1 ml of concentrated hydrochloric acid. The heptane reaction medium was evaporated under vacuum. The crude alpha-tocopherol was reacted with acetic anhydride (200 ml, 2.1 mole) for four hours at reflux temperature. The resulting acetic acid and excess acetic anhydride were removed under vacuum. The crude alpha-tocopherol acetate was assayed utilizing gas chromatography to determine the yield and purity. The results are shown in the table below.

Example 4

Example 3 is repeated except that a proportion of one weight percent of tridecylamine acetate, instead of octadecylamine acetate, is utilized based upon the weight of the crude isophytol used in the reaction.

Example 5

The procedure and proportions of Example 3 are repeated except that tridecylamine adipate is substituted for octadecylamine acetate.

Example 6

The procedure and proportions of Example 4 are repeated except that octadecylamine acetate is substituted for tridecylamine acetate.

Examples 7—10

The procedure and proportions of Example 4 are repeated except that the acetate salts of 1-docosanamine, eicosanamine, octadecyldimethyl amine, and dioctyldecylamine are substituted for tridecylamine acetate.

TABLE

| Example | Amine or amine salt | Amine level (wt. % to isophytol | Premixed and prereacted | Yield (% of theory) | Purity (%) |
|---|---|---|---|---|---|
| 1 (control) | Tridecyl-amine | 0.3 | + | 95.3 | 94.4 |
| 2 (control) | Tridecyl-amine | 1.0 | + | 95.7 | 94.6 |
| 3 | Octadecyl-amine acetate | 0.3 | − | 97.2 | 95.6 |

Example 11

Under a nitrogen atmosphere there were added to a reaction container 380 ml of technical grade n-heptane, 99.0 percent by weight trimethylhydroquinone (76.1 grams, 0.495 mole), 95 percent by weight zinc chloride (20.7 grams, 0.150 mole) and 0.3 weight percent of tridecylamine based upon the weight of the crude isophytol to be added subsequently, namely, 181 ml, (0.50 mole) of 97.5 percent isophytol. The mixture obtained was heated to constant reflux with a continuous hydrogen chloride sparge and then the isophytol was added over a 90 minute period. After completion of the isophytol addition, the hydrogen chloride sparge was continued for an additional 15 minutes, and five minutes later the heating was stopped. After cooling for a period of one hour, the reaction mixture was extracted with three portions (350 ml) of methanol/water (1:1) acidified with 1 ml of concentrated hydrochloric acid. The heptane reaction medium was evaporated under vacuum. The crude alpha-tocopherol was reacted with acetic anhydride (200 ml, 2.1 mole) for four hours at reflux temperature. The resulting acetic acid and excess acetic anhydride were removed under vacuum. The crude alpha-tocopherol acetate was assayed utilizing gas chromatography to determine the yield and purity. The results are shown in the table below.

Example 12

Example 11 was repeated except that a proportion of one weight percent of tridecylamine was utilized based upon the weight of the crude isophytol used in the reaction.

Example 13

The procedure and proportions of Example 11 were repeated except that octadecylamine was substituted for tridecylamine.

Example 14

The procedure and proportions of Example 12 were repeated except that octadecylamine was substituted for tridecylamine.

Examples 15—18

The procedure and proportions of Example 12 are repeated except that 1-docosanamine, eicosanamine, octadecyldimethyl amine, and dicotyldecylamine are substituted for tridecylamine.

TABLE

| Example | Amine | Amine level (wt. % to isophytol | Premixed and prereacted | Yield (% of theory) | Purity (%) |
|---|---|---|---|---|---|
| 1 (control) | Tridecyl-amine | 0.3 | + | 95.3 | 94.4 |
| 2 (control) | Tridecyl-amine | 1.0 | + | 95.7 | 94.6 |
| 11 | Tridecyl-amine | 0.3 | – | 96.5 | 94.8 |
| 12 | Tridecyl-amine | 1.0 | – | 96.8 | 94.9 |
| 13 | Octadecyl-amine | 0.3 | – | 98.2 | 95.4 |
| 14 | Octadecyl-amine | 1.0 | – | 97.0 | 95.4 |

**Claims**

1. A process for producing alpha tocopherol comprising reacting, in the presence of an inert organic solvent, trimethylhydroquinone with a phytyl derivative of the formula

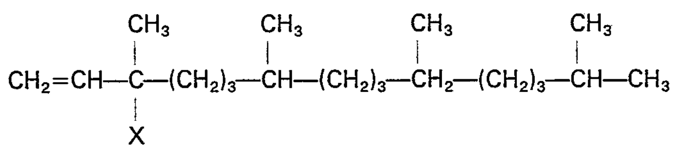

(I)

or

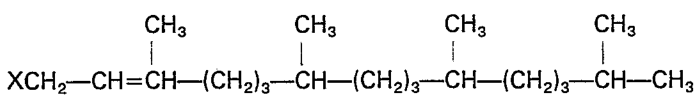

(II)

wherein X is a leaving group, in the presence of at least one Lewis acid, at least one strong acid, and an amine, characterized by adding to the reaction mixture prior to the addition of the phytyl derivative 0.05 percent to 5 percent by weight, based upon the weight of said phytyl derivative of an amine other than stearylamine and having 7 to 24 carbon atoms selected from the group consisting of primary, secondary or tertiary aliphatic, cycloaliphatic, or arylaliphatic amines or cyclohexylamine or a salt of said amine wherein said salt of said amine is the reaction product of a non-oxidizing protic acid having a pK of up to 5 and said amine.

2. The process of claim 1 wherein X is phenyl- or naphthyl sulfonyloxy, alkylsulfonyloxy with 1 to 7 carbon atoms, hydroxy, halogen, alkoxy with 1 to 7 carbon atoms, or alkanoyloxy with 1 to 7 carbon atoms.

3. The process of claim 2 wherein X is hydroxy.

4. The process of claim 1 wherein said amine is tridecylamine, octadecyldimethylamine, dioctyldecylamine, 1-docosanamine or 1-eicosanamine.

5. The process of claim 1 wherein said Lewis acid is boron trifluoride, boron tribromide, aluminum chloride, aluminum bromide, zinc chloride, or a boron trifluorophosphoric acid complex.

6. The process of claim 5 wherein said Lewis acid is zinc chloride and said protic acid is acetic acid.

7. The process of claim 5 wherein said strong acid is sulfuric acid, hydrochloric acid, hydrogen chloride gas, nitric acid, para-toluenesulfonic acid, or sodium bisulfate.

8. The process of claim 7 wherein said strong acid is hydrochloric acid.

9. The process of claim 7 wherein said strong acid is hydrogen chloride gas.

10. A process for producing alpha-tocopherol acetate comprising the process of producing alpha-tocopherol in accordance with the process of claim 2 and additionally reacting the product obtained with acetic anhydride.

**Patentansprüche**

1. Verfahren zur Herstellung von alpha-Tocopherol durch Umsetzen von Trimethylhydrochinon, in Gegenwart eines inerten organischen Lösungsmittels, mit einem Phytylderivat der Formel

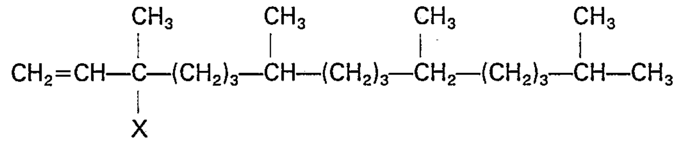

(I)

oder

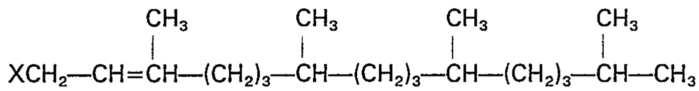

(II)

in der X eine Abgangsgruppe bedeutet, in Gegenwart mindestens einer Lewis-Säure, mindestens einer starken Säure und eines Amins, dadurch gekennzeichnet, daß man der Reaktionsmischung vor Zugabe des Phytylderivats 0,05 bis 5 Gewichtsprozen, bezogen auf das Gewicht des Phytylderivats, eines Aminsaußer Stearylamin—mit 7 bis 24 Kohlenstoffatomen, ausgewählt aus der Gruppe bestehend aus primären, sekundären und tertiären aliphatischen, cycloaliphatischen und arylaliphatischen Aminen, Cyclohexylamin und einem Salz des genannten Amins, wobei dieses Aminsalz das Reaktionsprodukt aus einer nichtoxidierenden Protonensäure mit einem pK-Wert von bis zu 5 und dem genannten Amin ist, zugibt.

2. Verfahren nach Anspruch 1, dadurch gekennzeichnet, daß X Phenyl- oder Naphthylsulfonyloxy, Alkylsulfonyloxy mit 1 bis 7 Kohlenstoffatomen, Hydroxy, Halogen, Alkoxy mit 1 bis 7 Kohlenstoffatomen oder Alkanoyloxy mit 1 bis 7 Kohlenstoffatomen bedeutet.

3. Verfahren nach Anspruch 2, dadurch gekennzeichnet, daß X Hydroxy bedeutet.

4. Verfahren nach Anspruch 1, dadurch gekennzeichnet, daß man als Amin Tridecylamin, Octadecyldimethylamin, Dioctyldecylamin, 1-Docosanamin oder 1-Eicosanamin verwendet.

5. Verfahren nach Anspruch 1, dadurch gekennzeichnet, daß man als Lewis-Säure Bortrifluorid, Bortribromid, Aluminiumchlorid, Aluminiumbromid, Zinkchlorid oder einen Bor-Trifluorphosphorsäure-Komplex verwendet.

6. Verfahren nach Anspruch 5, dadurch gekennzeichnet, daß man als Lewis-Säure Zinkchlorid und als Protonsäure Essigsäure verwendet.

7. Verfahren nach Anspruch 5, dadurch gekennzeichnet, daß man als starke Säure Schwefelsäure, Salzsäure, Chlorwasserstoffgas, Salpetersäure, para-Toluolsulfonsäure oder Natriumhydrogensulfat verwendet.

8. Verfahren nach Anspruch 7, dadurch gekennzeichnet, daß man als starke Säure Salzsäure verwendet.

9. Verfahren nach Anspruch 7, dadurch gekennzeichnet, daß man als starke Säure Chlorwasserstoffgas verwendet.

10. Verfahren zur Herstellung von alpha-Tocopherolacetat, in welchem man das Verfahren zur Herstellung von alpha-Tocopherol nach Anspruch 2 verwendet und zusätzlich das erhaltene Produkt mit Essigsäureanhydrid umsetzt.

**Revendications**

1. Procédé de production d'α-tocophérol, dans lequel on fait réagir, en présence d'un solvant organique inerte, de la triméthylhydroquinone avec un dérivé phytylique de formule

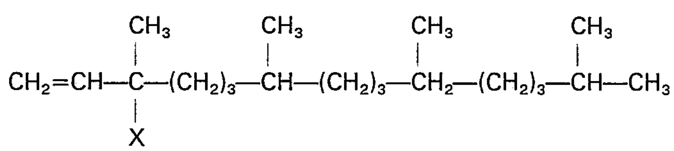

(I)

ou

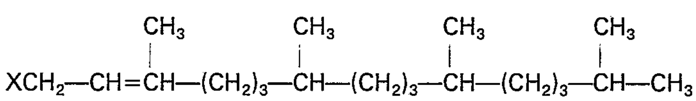

(II)

dans laquelle X est un groupe éliminable, en présence d'au moins un acide de Lewis, au moins un acide fort et une amine, caractérisé par le fait que l'on ajoute au mélange de réaction, avant l'addition du dérivé phytylique, 0,05% à 5% en poids, basé sur le poids du dit dérivé phytylique, d'une amine autre que la stéarylamine et ayant 7 à 24 atomes de carbone, choisie dans le groupe constitué des amines primaires, secondaires ou tertiaires aliphatiques, cycloaliphatiques, ou arylaliphatiques, ou cyclohexylamine ou un

sel de la dite amine, où ledit sel de ladite amine est le produit de réaction d'un acide protique non-oxydant, ayant un pK de jusqu'à 5, et de ladite amine.

2. Procédé selon la revendication 1, dans lequel X est phényl- ou naphtylsulfonyloxy, alkylsulfonyoxy de 1 à 7 atomes de carbone, hydroxy, halogène, alcoxy de 1 à 7 atomes de carbone ou alcanoyloxy de 1 à 7 atomes de carbone.

3. Procédé selon la revendication 2, dans lequel X est hydroxy.

4. Procédé selon la revendication 1 dans lequel ladite amine est tridécylamine, octadécyldiméthylamine, dioctyldécylamine, 3-docosanamine ou 1-eicosanamine.

5. Procédé selon la revendication 1, dans lequel ledit acide de Lewis est le trifluorure de bore, le tribromure de bore, le chlorure d'aluminium, le bromure d'aluminium, le chlorure de zinc ou un complexe bore acide trifluorophosphorique.

6. Procédé selon la revendication 5, dans lequel ledit acide de Lewis est le chlorure de zinc et ledit acide protique est l'acide acétique.

7. Procédé selon la revendication 5, dans lequel ledit acide fort est l'acide sulfurique, l'acide chlorhydrique, le gaz chlorhydrique, l'acide nitrique, l'acide paratoluènesulfonique ou le bisulfate de sodium.

8. Procédé selon la revendication 7 dans lequel ledit acide fort est l'acide chlorhydrique.

9. Procédé selon la revendication 7, dans lequel ledit acide fort est le gaz chlorhydrique.

10. Procédé de préparation d'acétate d'α-tocophérol dans lequel on produit de l'α-tocophérol selon le procédé de la revendication 2 et ensuite on fait réagir le produit obtenu avec de l'anhydride acétique.